# EUROPEAN PATENT APPLICATION

(11) **EP 4 450 065 A1**
(43) Date of publication of application: **23.10.2024**
(21) Application number: 24170171.3
(22) Date of filing: 15.04.2024
(51) Int. Cl.: A61K 9/127

(54) **LIPOSOMAL COMPOSITION COMPRISING IRON SULPHATE FOR USE IN THE TREATMENT AND PREVENTION OF ANAEMIA IN PREGNANT WOMEN AND DURING PUERPERIUM**

(30) Priority: 19.04.2023 IT 202300007608
(71) Applicant: Bio-Therapic Italia S.r.l., 00193 Roma (IT)
(72) Inventor: INFORTUNA, Paolo, I-00193 Roma (IT); POLLICHIENI, Pietro, I- 00193 Roma (IT)
(74) Representative: Long, Giorgio

(57) **Abstract**

The present invention relates to a liposomal composition containing an iron(II) salt, in particular iron sulphate, for use in the treatment and prevention of anaemia in pregnant women and during puerperium.

## Description

### Fild of the invention

The present invention relates to a liposomal composition containing an iron(II) salt, in particular iron sulphate, for use in the treatment and prevention of anaemia in pregnant women and during puerperium.

### Background of the invention

As is known, iron plays an essential role in energy metabolism and in the synthesis of molecules indispensable for life, in particular the "heme" group that is part of the structure of haemoglobin, responsible for oxygen transport in the blood. However, the reduced availability of iron when taken in the form of salts or through food is also known, as well as its high toxicity linked to the pro-oxidant effect it can exhibit when taken in excess.

Iron deficiency can be linked to a specific pathology (iron-deficiency anaemia), or simply to a momentary or transitory condition (for example, pregnant women), making the population needing to supplement their iron deficiency with an external intake rather extensive.

The use of orally administered iron-based formulations is generally considered first-line treatment for iron deficiency as they are more economical compared to those for intravenous use and of quicker and more convenient access for the patient. Classic indications recommend 100-200 mg of elemental iron per day, away from meals. However, subjects undergoing treatment with orally administered iron-based preparations, have been found to experience a prevalence of adverse effects, mainly of gastrointestinal nature (nausea, constipation or diarrhoea), of about 12%. Additional issues inherent to an oral administration of iron are the failure to correct the deficiency (poor treatment efficacy) and a generalized inflammatory state that suggests a lack of response due to high levels of circulating hepcidin. To overcome such problems, intravenous administration is therefore suggested. This type of administration must necessarily take place in a hospital setting, resulting in discomfort for the patient.

In the context of intravenous therapy, intravenous treatment with iron gluconate resulted in a correction of iron-deficiency anaemia similar to that obtained with oral iron sulphate and a similar reduction of renal function over the 24-month study period. Nevertheless, the study was prematurely terminated due to a significant increase of severe adverse events of cardiovascular and infective nature. Vice versa, it has been demonstrated that the use of ferric carboxymaltose (FCM) for 24 weeks in patients with congestive heart failure (NYHA II or III), restoring iron stores, determines an improvement in symptoms and NYHA functional class (primary endpoints) as well as cognitive and physical performance (6-minutes walk test) and quality of life (secondary endpoints), in the absence of significant side effects. However, such intravenous treatment still presents the problem of needing to be administered in a hospital setting and of causing considerable discomfort to the patient.

In pregnant women, anaemia is defined by the detection of haemoglobin (Hgb) levels lower than 11 g/dl in the first trimester or lower than 10,5 g/dl in the second trimester, or lower than 11 g/dl in the third trimester. Anaemia can be acquired, such as deficiency anaemia (e.g. iron, vitamin B12, folates), haemorrhagic anaemia, anaemia from chronic diseases, aplastic or hereditary anaemia, such as thalassemia, sickle cell anaemia or haemoglobinopathies other than sickle cell anaemia. Iron deficiency anaemia is extremely common during pregnancy. It is an acquired anaemia characterized by increased production of red blood cells with mean corpuscular volume (MCV) lower than 80 fl (microcytic anaemia). A national study on anaemia during pregnancy found a prevalence of 22 pregnant women out of 1,000 in the United States.

The aim of the present invention is therefore to provide a treatment for anaemia in pregnant women suffering from anaemia, in particular mild anaemia, that is effective, substantially free of side effects and easily administered also by the patient itself.

A further aim of the invention is to provide a method for preventing postpartum anaemia in puerperal women, in particular in women who had shown anaemia during pregnancy.

### Summary of the invention

An object of the present invention is therefore a liposomal composition comprising liposomes containing iron (II) sulphate and vitamin C, for use in the treatment and prevention of anaemia in pregnant women.

Another object of the invention is a liposomal composition comprising liposomes containing iron(II) sulphate and vitamin C, for use in preventing anaemia in puerperal women.

### Detailed description of the invention

According to a first object, the invention relates to a liposomal composition comprising liposomes containing iron (II) sulphate and vitamin C, for use in the treatment and prevention of anaemia in pregnant women.

According to a further object of the invention, the invention relates to a liposomal composition comprising liposomes containing iron(II) sulphate and vitamin C, for use in preventing anaemia in puerperal women.

Liposomes used for the purposes of the present invention are for example those described in the Italian Patent No. IT 1269946 granted on 16/04/1997 (application No. 101994900375993 of 24/06/1995) .

Liposomes according to the invention form a liposomal component composed of phospholipid vesicles, for example lecithin phospholipids, containing per dose unit:
- 20-40 mg iron(II) sulphate;
- 20-30 mg vitamin C.

The composition according to the invention, in addition to said liposomal component, comprises, per dose unit, 300-400 mg maltodextrins.

In a preferred embodiment, the composition according to the invention comprises, per dose unit:
- liposomes containing 30 mg iron(II) sulphate (150 mg 20% solution) and 24 mg vitamin C;
- 335 mg maltodextrins.

The composition may be presented in the form of granulate in enteric capsules and may further contain other non-active excipients such as magnesium stearate and silicon dioxide.

The composition of the invention can also be administered in the form of pharmaceuticals, dietary products, food supplements and foodstuff for special medical purposes (AEMS).

The term "foodstuff for special medical purposes" shall mean products authorized according to Regulation (UE) 2016/128. This term refers to a product to be administered under medical supervision, thus assimilating such AFMSs to a drug.

### EXPERIMENTAL PART

In this study, women who received oral supplementation with the composition of the invention as treatment for mild iron deficiency anaemia were compared with women who did not receive it. The treated group received 30 mg of iron sulphate along with liposomal vitamin C (composition of the invention as defined above) once daily starting from the diagnosis of mild anaemia until delivery. Only those who started treatment before the 28th week of gestation were included in the study.

The control group consisted of women with mild anaemia who had not received iron therapy. Inclusion criteria were singleton pregnancy with mild microcytic iron deficiency anaemia. Mild anaemia was defined as anaemia with a level of Hgb between 9,00 g/dl and 10,99 g/dl. The primary outcome of the study was the determination of the incidence of anaemia after treatment. Secondary outcomes were the increase of serum ferritin, the increase of Hgb levels and maternal tolerance. Primary and secondary outcomes were compared after two months of daily administration of the composition and after two months of follow-up after the delivery.

### Statistical analysis

Statistical analysis was carried out using Statistical Package for Social Sciences (SPSS) v. 19.0 (IBM Inc., Armonk, NY, USA). Data was presented as means ± standard deviation or as number (percentage). Dichotomous data was compared with the chi-square test. The between groups comparisons were made using the T test to test group means assuming equal variances within the group.

### Results

In the study were included 200 women, 100 for each group. Twelve women were lost to follow-up. Therefore, 188 participants were available for the final analysis. The two groups were similar in terms of maternal demographic data: both had similar gestational anaemia and with similar phenotype. Maternal age and also the rate of smokers were similar. The women receiving the treatment showed a significantly lower overall incidence of anaemia after 2 months and at the postpartum follow-up visit. Further, the increase of Hgb level and serum ferritin was significantly higher in treated women. Maternal tolerance was adequate in the treatment group.

The data is reported in table 1.

**Table 1: primary and secondary results**

| | Treated women | Control group | P-value |
|---|---|---|---|
| | N=88 | N=100 | |
| anaemia | | | |
| 2 months follow-up | 13 | 38 | <0.01 |
| anaemia | | | |
| 2 months postpartum | 7 | 21 | <0.01 |
| Hgb level | +1.9±0.7 | +0.4±1.1 | 0.03 |
| Ferritin level | 19±4.8 | 6±3.7 | 0.04 |

All data is statistically significant.

The study has demonstrated that treating pregnant women with the composition of the invention is safe and effective in reducing the recurrence of anaemia.

The group of treated women, i.e. singleton pregnancy with mild iron deficiency anaemia, is the one at greatest risk of anaemia and postpartum haemorrhages.

### FORMULATION EXAMPLE

Vegetable gelatine capsules containing:
- liposomes containing 30 mg iron sulphate and 24 mg vitamin C
- 335 mg maltodextrins
- 10 mg magnesium stearate
- 5 mg silicon dioxide.

## Claims

1. A liposomal composition comprising liposomes containing iron(II) sulphate and vitamin C, for use in the treatment and prevention of anaemia in pregnant or in puerperal women.

2. The composition for use according to claim 1, wherein the treatment is administered before the 28th week of gestation.

3. The composition for use according to claim 1 or 2, wherein the liposomes form a liposomal component consisting of phospholipid vesicles, preferably lecithin phospholipids, containing per dose unit:
- 20-40 mg iron(II) sulphate;
- 20-30 mg vitamin C.

4. The composition for use according to claim 3, wherein, in addition to the aforesaid liposomal component, the composition comprises, per dose unit, 300-400 mg maltodextrins.

5. The composition for use according to any one of claims 1 to 4, wherein the composition, per dose unit, comprises or consists of:
- liposomes containing 30 mg iron(II) sulphate (150 mg 20% solution) and 24 mg vitamin C;
- 335 mg maltodextrins;
- non-active excipients.

6. The composition for use according to any one of claims 1 to 5, wherein said composition induces an increase in haemoglobin and ferritin levels.

7. The composition for use according to any one of claims 1 to 6, wherein said composition is formulated as a pharmaceutical, dietary product, food supplements and foodstuff for special medical purposes (FSMPs).
